# EUROPEAN PATENT SPECIFICATION

(11) **EP 3 459 499 B1**
(45) Date of publication and mention of the grant of the patent: **24.08.2022**
(21) Application number: 18195878.6
(22) Date of filing: 21.09.2018
(51) Int. Cl.: A61F 2/24

(54) **PROSTHETIC HEART VALVE WITH ATRAUMATIC AORTIC PORTION**
HERZKLAPPENPROTHESE MIT ATRAUMATISCHEM AORTENABSCHNITT
VALVULE CARDIAQUE PROTHÉTIQUE À PARTIE AORTIQUE ATRAUMATIQUE

(30) Priority: 22.09.2017 US 201762561909 P
(43) Date of publication of application: 27.03.2019
(73) Proprietor: St. Jude Medical, Cardiology Division, Inc., St. Paul, MN 55117 (US)
(72) Inventor: ALKHATIB, Yousef F., Edina, MN 55439 (US); KALETA, Richard, Arden Hills, MN 55112 (US); ERZBERGER, Gary, Plymouth, MN 55442 (US)
(74) Representative: Gill Jennings & Every LLP

(56) References cited:
- WO-A1-2015/126711
- WO-A1-2018/039543
- WO-A2-2009/042196
- US-A1- 2012 271 398
- US-A1- 2013 150 956
- US-A1- 2015 148 893

## Description

The present inventions relate to heart valve replacement and, in particular, to collapsible prosthetic heart valves. More particularly, the present inventions relate to repositionable collapsible prosthetic heart valves.

Prosthetic heart valves that are collapsible to a relatively small circumferential size can be delivered into a patient less invasively than valves that are not collapsible. For example, a collapsible valve may be delivered into a patient via a tube-like delivery apparatus such as a catheter, a trocar, a laparoscopic instrument, or the like. The ability of the valve to collapse can avoid the need for a more invasive procedure such as full open-heart surgery via a sternotomy.

Collapsible prosthetic heart valves typically take the form of a valve structure mounted on a stent. There are two types of collapsible stents on which the valve structures are ordinarily mounted: a self-expanding stent and a balloon-expandable stent. To place such valves into a delivery apparatus and ultimately into a patient, the valve must first be collapsed or crimped to reduce its circumferential size.

When a collapsed prosthetic valve has reached the desired implant site in the patient (*e.g.,* at or near the annulus of the patient's heart valve that is to be replaced by the prosthetic valve), the prosthetic valve can be deployed or released from the delivery apparatus and re-expanded to full operating size. For balloon-expandable valves, this generally involves releasing the entire valve, assuring its proper location, and then expanding a balloon positioned within the valve stent. For self-expanding valves, on the other hand, the stent automatically expands as the sheath covering the valve is withdrawn.

Despite the various improvements that have been made to the collapsible prosthetic heart valve delivery process, conventional delivery devices, systems, and methods suffer from some shortcomings. Therefore, a need exists for further improvements to collapsible prosthetic heart valves, and in particular, self-expanding prosthetic heart valves. Among other advantages, the present inventions may address one or more of these needs. WO 2015/126711, WO 2009/042196, US 2015/148893 and WO 2018/039543 all disclose prosthetic heart valves.

The present invention provides a prosthetic heart valve for replacing a native valve, the prosthetic heart valve comprising: a stent having an inflow end, an outflow end, are an annulus section adjacent the inflow end, an aortic section adjacent the outflow end, a transition section between the annulus section and the aortic section, a collapsed condition and an expanded condition, the stent including a first circumferential row of cells and a second circumferential row of cells positioned closer to the outflow end of the stent than the first circumferential row; a first cuff attached against a surface of the stent, the first cuff having an inflow edge and an outflow edge, the inflow edge being substantially straight, and the outflow edge including a series of first peaks extending a first distance toward the outflow end of the stent alternating with a series of second peaks extending a second distance toward the outflow end of the stent, the second distance being less than the first distance, the outflow edge of the first cuff being smoothly curved between each of the first peaks and each adjacent second peak so that a portion of the first cuff between the first peak and an adjacent second peak partially covers a cell in the second circumferential row; a second cuff having an inflow end and outflow end, the second cuff being located radially outward of the first cuff and radially outward of the stent, the inflow end of the second cuff having a substantially straight edge and the outflow end of the second cuff having an undulating edge; and a valve assembly arranged radially inward of the first cuff.

An aspect of the present disclosure provides a further embodiment of a prosthetic heart valve for replacing a native valve. The prosthetic heart valve includes a stent having an inflow end, an outflow end, an annulus section adjacent the inflow end, an aortic section adjacent the outflow end, a transition section between the annulus section and the aortic section, a collapsed condition and an expanded condition, the stent including a first circumferential row of cells and a second circumferential row of cells positioned closer to the outflow end of the stent than the first circumferential row, each of the cells in the first circumferential row of cells being connected to an adjacent cell in the first circumferential row at a connection point, each of the connection points being positioned at a first distance from the inflow end of the stent, the inflow end of the stent being curved radially inward so that, in the expanded condition, a diameter of the stent at the inflow end of the stent is smaller than a diameter of the stent at the first distance from the inflow end of the stent; and a valve assembly arranged within the stent, the valve assembly including a cuff and a plurality of leaflets.

### BRIEF DESCRIPTION OF THE DRAWINGS

Various embodiments of the presently disclosed delivery system are disclosed herein with reference to the drawings, wherein:
FIG. 1 is a side elevational view of a prior art collapsible prosthetic heart valve;
FIG. 2 is a schematic cutaway view of the heart, showing two approaches for delivering a prosthetic aortic heart valve;
FIG. 3 is a side elevational view of one embodiment of a collapsible prosthetic heart valve according to the present disclosure;
FIGS. 4A-B are schematic developed views of cuffs for collapsible prosthetic heart valves;
FIG. 5 is a partial side elevational view of the annulus section of a collapsible prosthetic heart valve having an inner cuff and an outer cuff according to the present disclosure;
FIG. 6 is an enlarged view of a bottom portion of the annulus section of the collapsible prosthetic heart valve of FIG. 5;
FIG. 7 is an enlarged schematic view showing details of the stent structure in Regions A and B of the prosthetic heart valve shown in FIG. 3;
FIG. 8 is an enlarged schematic view showing details of the stent structure in Regions C and D of the prosthetic heart valve shown in FIG. 3;
FIG. 9 is an enlarged view of the aortic section of a stent having an inward taper;
FIG. 10 is an enlarged schematic view of one example of a commissure attachment feature of a stent; and
FIG. 11 is a side elevational view of another embodiment of a collapsible prosthetic heart valve according to the present disclosure.

Various embodiments of the present invention will now be described with reference to the appended drawings. It will be appreciated that these drawings depict only some embodiments of the invention and are therefore not to be considered limiting of its scope.

### DETAILED DESCRIPTION OF THE INVENTION

As used herein, the term "proximal," when used in connection with a prosthetic heart valve, refers to the end of the heart valve closest to the heart when the heart valve is implanted in a patient, whereas the term "distal," when used in connection with a prosthetic heart valve, refers to the end of the heart valve farthest from the heart when the heart valve is implanted in a patient. When used in connection with devices for delivering a prosthetic heart valve into a patient, the terms "trailing" and "leading" are to be taken as relative to the user of the delivery devices. "Trailing" is to be understood as relatively close to the operator, and "leading" is to be understood as relatively farther away from the operator.

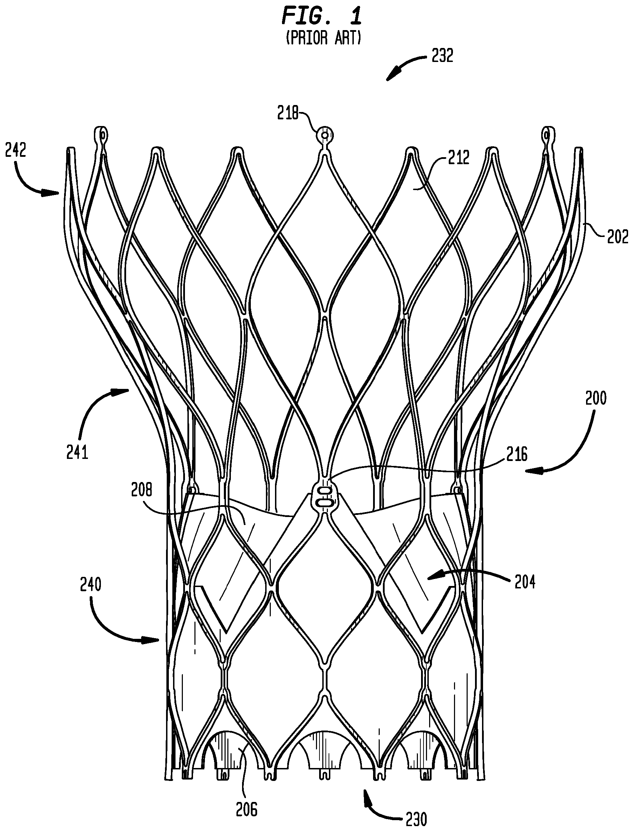

FIG. 1 shows a collapsible prosthetic heart valve 200 according to the prior art. The prosthetic heart valve 200 is designed to replace the function of the native aortic valve of a patient. Examples of collapsible prosthetic heart valves are described in International Patent Application Publication No. WO/2009/042196; United States Patent No. 7,018,406; and United States Patent No. 7,329,27 8.

Prosthetic heart valve 200 includes an expandable stent 202 which may be formed from biocompatible materials that are capable of self-expansion, such as, for example, shape memory alloys such as nitinol. Stent 202 extends from a proximal or inflow end 230 to a distal or outflow end 232, and includes an annulus section 240 adjacent the proximal end and an aortic section 242 adjacent the distal end. The annulus section 240 has a relatively small circumference in the expanded condition, while the aortic section 242 has a relatively large circumference in the expanded condition. Preferably, annulus section 240 is in the form of a cylinder having a substantially constant diameter along its length. A transition section 241 tapers outwardly from the annulus section 240 to the aortic section 242. Each of the sections of the stent 202 includes a plurality of cells 212 connected to one another in one or more annular rows around the stent, each of the cells being formed by a plurality of struts. For example, as shown in FIG. 1, the annulus section 240 may have two annular rows of complete cells 212 and the aortic section 242 and transition section 241 may each have one or more annular rows of partial cells 212. The cells 212 in the aortic section 242 may be larger than the cells 212 in the annulus section 240. The larger cells in the aortic section 242 better enable the prosthetic valve 200 to be positioned without the stent structure interfering with blood flow to the coronary arteries.

Stent 202 may include one or more retaining elements 218 at the distal end 232 thereof, the retaining elements being sized and shaped to cooperate with female retaining structures provided on the deployment device (not shown). The engagement of retaining elements 218 with the female retaining structures on the deployment device helps maintain prosthetic heart valve 200 in assembled relationship with the deployment device, minimizes longitudinal movement of the prosthetic heart valve relative to the deployment device during unsheathing or resheathing procedures, and helps prevent rotation of the prosthetic heart valve relative to the deployment device as the deployment device is advanced to the target location and during deployment.

The stent 202 may also include a plurality of commissure attachment features (CAFs) 216 for attaching the commissure between two adjacent leaflets to the stent. As can be seen in FIG. 1, CAFs 216 may lie at the intersection of four cells 212, two of the cells being adjacent one another in the same annular row, and the other two cells being in different annular rows and lying in end-to-end relationship. Preferably, CAFs 216 are positioned entirely within annulus section 240 or at the juncture of annulus section 240 and transition section 241. CAFs 216 may include one or more eyelets which facilitate the suturing of the leaflet commissure to the stent.

The prosthetic heart valve 200 includes a valve assembly 204 positioned in the annulus section 240. Valve assembly 204 may be secured to stent 202 by suturing to the struts constituting the cells of the stent and/or suturing to the CAFs 216 of the stent. Valve assembly 204 includes a cuff 206 and a plurality of leaflets 208 which collectively function as a one-way valve by coapting with one another. FIG. 1 illustrates a prosthetic heart valve for replacing a native tri-leaflet valve, such as the aortic valve. Accordingly, prosthetic heart valve 200 is shown in FIG. 1 with three leaflets 208, as well as three CAFs 216. However, it will be appreciated that each of the prosthetic heart valves described herein may have a greater or lesser number of leaflets and CAFs.

Although cuff 206 is shown in FIG. 1 as being located on the lumenal or inner surface of annulus section 240, it is contemplated that the cuff may be located on the ablumenal or outer surface of the annulus section, or may cover all or part of either or both of the lumenal and ablumenal surfaces of the annulus section. Both the cuff 206 and the leaflets 208 may be wholly or partly formed of any suitable biological material, such as bovine or porcine pericardium, or biologically acceptable polymers, such as PTFE, urethanes and the like.

As is shown in FIG. 1, the entirety of valve assembly 204, including the leaflet commissures, is positioned in the annulus section 240 of stent 202. When open, the leaflets may extend further into the transition section 241 or may be designed such that they remain substantially completely within the annulus section 240. That is, in this particular heart valve, substantially the entirety of valve assembly 204 is positioned between the proximal end 230 of stent 202 and CAFs 216, and none of the valve assembly is positioned between the CAFs and the distal end 232 of the stent.

The prosthetic heart valve described above may be used to replace a native heart valve, such as the aortic valve, a surgical heart valve or a heart valve that has undergone a surgical procedure. The prosthetic heart valve may be delivered to the desired site (*e.g.,* near the native aortic annulus) using any suitable delivery device. During delivery, the prosthetic heart valve is disposed inside the delivery device in the collapsed condition. The delivery device may be introduced into a patient using a transfemoral, transapical, transaortic, subclavian, or transseptal approach. Once the delivery device has reached the target site, the user may deploy the prosthetic heart valve. Upon deployment, the prosthetic heart valve expands into secure engagement within the native anatomic structure such as the aortic annulus. When the prosthetic heart valve is properly positioned inside the patient, it works as a one-way valve, allowing blood to flow in one direction and preventing blood from flowing in the opposite direction.

In a prosthetic heart valve, the valve assembly may be spaced from the distal or aortic end of the stent by a distance that enables deployment of the heart valve by an amount sufficient for the valve leaflets of the prosthetic valve to operate as intended, while the distal end of the stent remains captured by the delivery device. More particularly, as will be explained further below, the annulus end of the prosthetic heart valve may be deployed first while the aortic end of the prosthetic heart valve remains at least partially covered by the sheath of the delivery device. The annulus portion of the prosthetic heart valve may be deployed so that the entirety of the valve leaflets, up to and including the commissures, is deployed and fully operational. By deploying the prosthetic heart valve in this manner, the user can determine whether the valve leaflets are properly positioned relative to the native valve annulus, and whether the valve is functioning properly. If the user determines that the positioning and operation of the valve are acceptable, the remainder of the valve may be deployed. However, if it is determined that the leaflet position is improper or that the valve is not functioning properly, the user may resheath the valve and either reposition it for redeployment, or remove it entirely from the patient. This can be particularly important in very high-risk patients who would typically be recipients of these types of valves, because of the nature of their condition and the impact that may have on the shape and/or condition of the native valve and valve annulus.

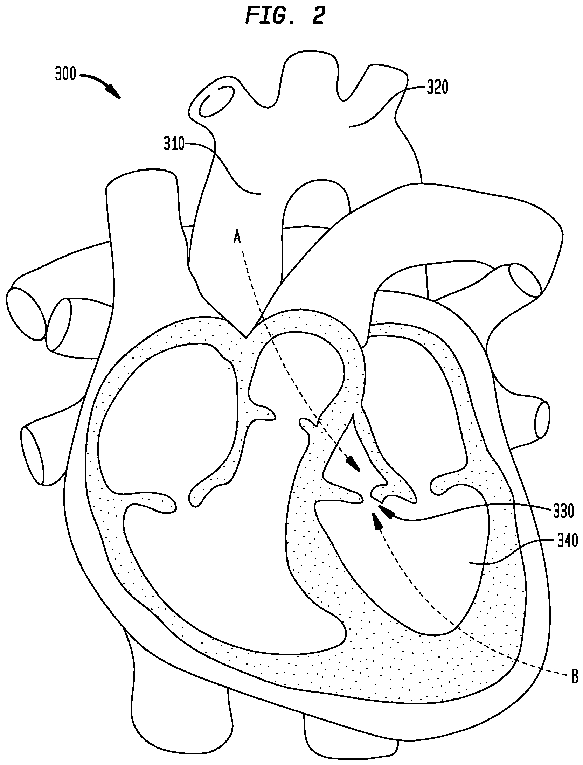

FIG. 2 illustrates a human heart 300 and two different approaches for delivering a prosthetic heart valve to its intended target at the aortic valve 330. As illustrated in FIG. 2, the heart 300 includes an aorta 310, an aortic arch 320 and a left ventricle 340. At least two separate paths are available for introducing a prosthetic heart valve to the aortic valve 330.

A transfemoral approach is indicated by the dashed arrow "A" in FIG. 2. In this method, the prosthetic heart valve is inserted into the femoral artery, tracked through the vasculature and then introduced to the target site via the aortic arch 320. Echocardiography and other means may be used to help guide the delivery device through this approach.

A second dashed arrow "B" indicates a transapical approach for delivering the prosthetic heart valve. In transapical delivery, a small incision is made between the ribs and then into the apex of the left ventricle 340. The prosthetic heart valve is delivered directly to the target site through these incisions.

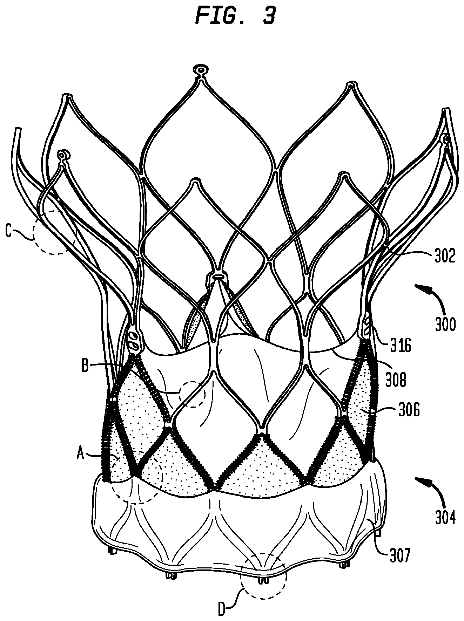

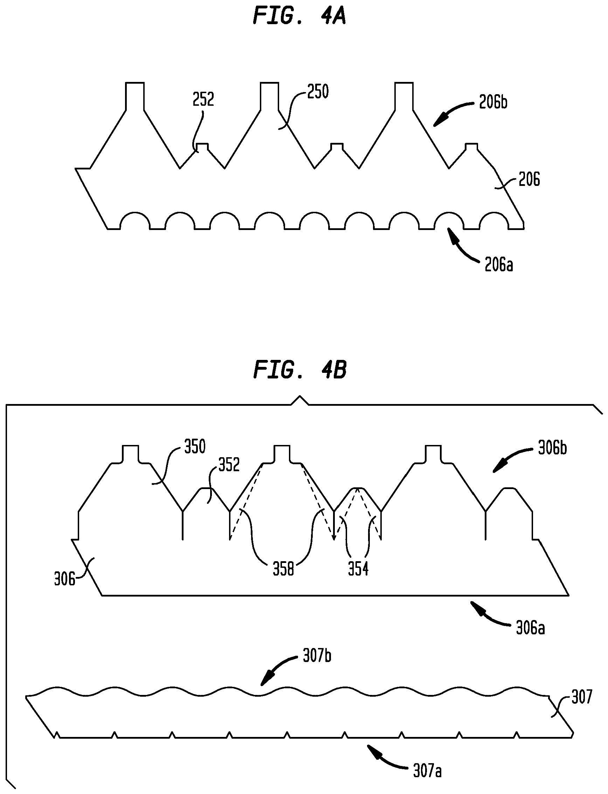

FIG. 3 is a side elevational view of one embodiment of a collapsible prosthetic heart valve 300 according to the present disclosure. Several of the specific differences between prosthetic heart valve 300 and prosthetic heart valve 200 will be described with reference to FIGS. 4-10. Generally, prosthetic heart valve 300 includes many of the same components as prosthetic heart valve 200, including a stent 302 and a valve assembly 304 having an inner cuff 306 and leaflets 308. Inner cuff 306 may include a fabric formed of a plurality of fibers, including first and second sets of fibers that are woven together, the fabric being oriented such that the first set of fibers extend at an angle to the longitudinal axis of stent 302, as will be described with reference to FIG. 4B.

Prosthetic heart valve 300 further includes an outer cuff 307 located near the annulus section of the stent and covering the bottom half of the lowermost row of cells. Inner cuff 306 and outer cuff 307 may be partially or fully formed of the same material (e.g., the same fabric).

Stent 302 may be sized to accommodate a range of native annulus diameters. In at least some embodiments, stent 302 may be provided in four sizes, each with a range chosen to accommodate one of the following: (1) a native annulus having a 19-21 mm diameter, (2) a native annulus having a 21-23 mm diameter, (3) a native annulus having a 23-25 mm diameter, and (4) a native annulus having a 25-27 mm diameter. These four sizes expand the use range when compared to other traditional heart valves. Likewise, stent 302 may be sized to accommodate a range of aorta diameters. In at least some embodiments, stent 302 may be provided in one of the following aortic use ranges, each corresponding to one of the annulus sizes listed above: (1) aortic use range of 26-36 mm, (2) aortic use range of 28-38 mm, (3) aortic use range of 30-40 mm, and (4) aortic use range of 32-42 mm. These four examples allow the prosthetic heart valve of the present disclosure to be used for patients with smaller annulus to aortic ratios.

FIGS. 4A-B show some configurations of cuffs for use with collapsible prosthetic heart valves. Specifically, FIG. 4A shows a traditional cuff 206. Cuff 206 has a scalloped inflow end 206a and a zig-zag outflow end 206b including a series of tall peaks 250 that extend up to CAFs 216 alternating with a series of shorter or mid-peaks 252.

FIG. 4B shows an ultra high molecular weight polyethylene (UHMWPE) fabric for the inner cuff 306, and an ultra high molecular weight polyethylene (UHMWPE) fabric for the outer cuff 307. Inner cuff 306 has a shape that is similar to that of cuff 206, with a primary difference being that inner cuff 306 has a substantially straight inflow edge 306a rather than a scalloped inflow edge. The outflow edge 306b of inner cuff 306 includes a series of tall peaks 350 that extend up to CAFs 316 alternating with a series of shorter or mid-peaks 352. At the intersections between each tall peak 350 and each mid-peak 352, inner cuff 306 may include a slit 354 that extends part of the way toward inflow edge 306a in a direction substantially orthogonal to the inflow edge. Slits 354 enable side portions 356 of mid-peaks 352 to be folded around certain struts of the valve stent, as described more fully below. Slits 354 also enable side portions 358 of tall peaks 350 to be folded against the outer surface of inner cuff 306 before attaching the cuff to the valve stent. Outer cuff 307, on the other hand, is shorter in height than inner cuff 306, with a substantially straight inflow edge 307a and an undulating outflow edge 307b. These configurations may reduce the profile of the crimped valve to minimize the forces required to load the prosthetic heart valve into a delivery device, and, at the same time, decrease the leakage of blood around the outside of an implanted valve, often referred to as paravalvular leakage. Additionally, the use of the UHMWPE material may enable the elimination of an underwire at the attachment locations of the leaflets to the inner cuff due to the strength of the cuff, further decreasing the crimped profile of the heart valve. As previously mentioned, the fabric forming inner cuff 306 may include first and second sets of fibers woven together. In at least some examples, one of the sets of fibers (*e.g.,* the first set of fibers) is oriented at an angle a1 with respect to the longitudinal axis of stent 302. In some examples, angle a1 may be between about 30 degrees and 60 degrees with respect to the longitudinal axis of stent 302; in other examples, angle a1 may be between about 40 degrees and 50 degrees with respect to the longitudinal axis of stent 302. In still other examples, angle a1 may be about 45 degrees with respect to the longitudinal axis of stent 302.

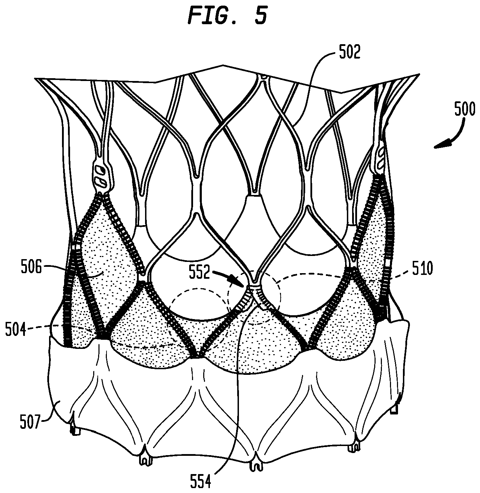

FIG. 5 is a partial side elevational view of the annulus portion of a collapsible prosthetic heart valve 500 according to the invention. . As shown in FIG. 5, side portions 554 of the mid-peaks 552 of inner cuff 506, similar to those of inner cuff 306, are folded over certain struts of stent 502 at region 510 to reduce abrasion of the leaflet on the cuff. Additionally, rather than having a sharp "V"-shaped valley between the peaks of the outflow edge of the cuff, as in cuffs 206 and 306, inner cuff 506 is smoothly curved between adjacent peaks, providing an extra landing zone region 504 to improve sealing in certain conditions. The outer cuff 507 of prosthetic heart valve 500 is similar in shape to outer cuff 307 described above. As shown, the peaks of the undulating outflow edge 3076 are connected to the stent struts at the intersection of adjacent cells in the first row of cells at the inflow end of the stent, while the valleys of the undulating outflow edge are not connected to the stent or to inner cuff 306, but rather are free to flex radially outward.

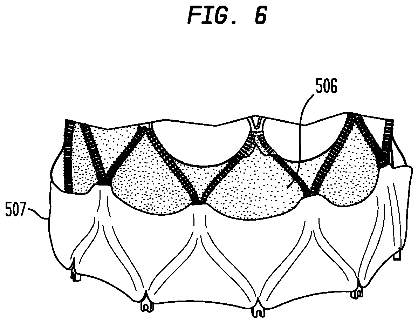

FIG. 6 is an enlarged view of a bottom portion of the annulus section of a collapsible prosthetic heart valve 500. This portion shows the inflow edge of inner cuff 506 and outer cuff 507. As shown, the inflow edges include no cutouts adjacent the lowermost row of cells of the stent as opposed to the scalloped inflow edge of cuff 206 of FIG. 1. This may improve sealing at the base of the prosthetic heart valve.

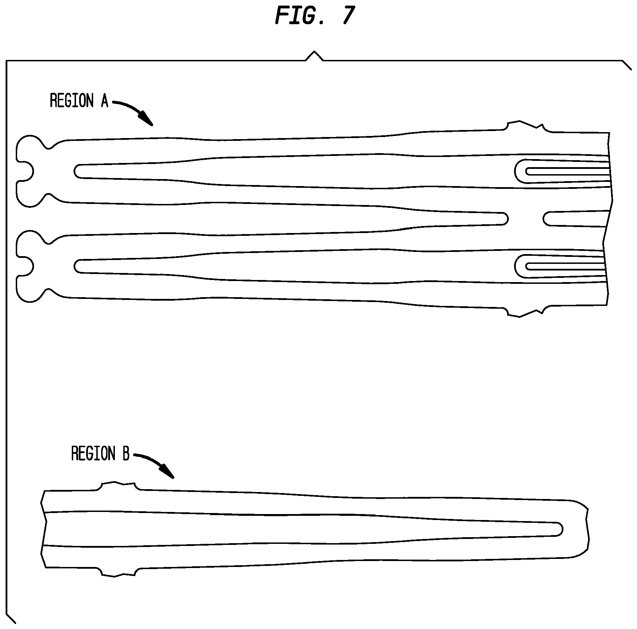

FIG. 7 is an enlarged schematic view showing several distinctions in stent 302, and specifically the differences in the stent in regions A and B of FIG. 3. As shown in FIG. 7, no runner is formed between adjacent cells in region A, such that the cells in the second full row of cells are directly connected to the bottom row of struts. This structure is provided not only in region A, but throughout stent 302, with the exception of the annular row of cells that include CAFs 316. This may reduce delivery system forces and maintain the overall length of the stent.

In a second difference, shown in region B, each of the struts in the annulus section of the stent may be slightly tapered from a larger cross-section at the ends of the struts to a smaller cross-section intermediate the ends. The tapering of the struts in the annulus section of the stent may reduce delivery system forces and reduce the dilation of the delivery system sheath after loading, while maintaining the outward radial forces exerted by the stent upon deployment.

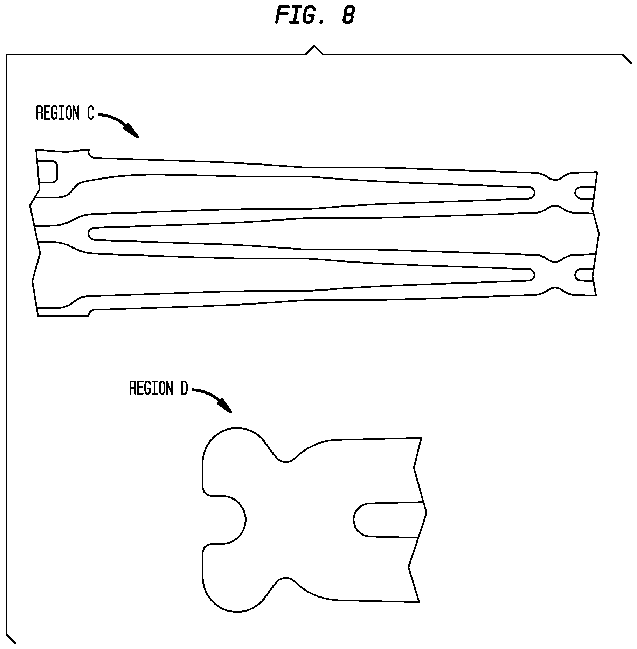

FIG. 8 is an enlarged schematic view showing some additional distinctions in stent 302. FIG. 8 again shows a lack of runners between adjacent cells in region C. In addition, an aortic hinge feature is added to maintain tractability of the stent in the delivery system. The aortic hinge is formed by tapering all or most of the struts in the aortic section of the stent at a predetermined distance from the outflow end of the stent. As a result of this tapering, the stent has increased flexibility at this predetermined distance, and is more easily able to flex in any direction as the prosthetic heart valve is advanced through the patient's vasculature to the implantation site. Horseshoes in region D are also shown which accommodate two sutures instead of one to accommodate two different cuffs. While the horseshoe shown in FIG. 8 is different from those shown in other figures, it is contemplated that either horseshoe configuration can be incorporated in any of the stents described above. Although FIGS. 7 and 8 show a lack of runners in regions A and C, it is contemplated that, in certain embodiments hereof, runners similar to those shown in FIGS. 1 and 3 may be formed between adjacent cells in region A, region C, and between cells elsewhere in stent 302.

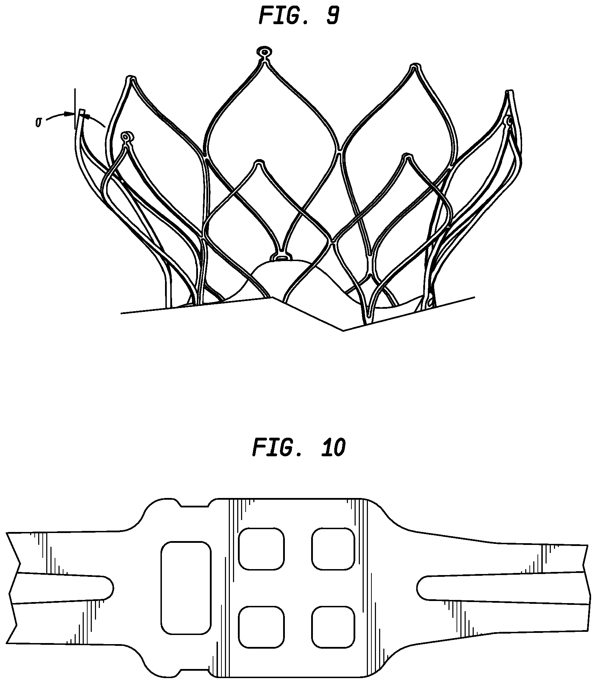

FIG. 9 shows an inward curve σ at the outflow end of the stent to reduce the risk of distal cells perforating or damaging the aorta. In some examples, the outflow end of the aortic section is inwardly slanted from a longitudinal axis of the stent by between 5 and 15 degrees. In other examples, the aortic section is inwardly slanted from the longitudinal axis of the stent by between 8 and 12 degrees, or by about 10 degrees.

FIG. 10 shows tapers at certain portions of the commissure attachment feature 316 to reduce strain for improved fatigue performance. Although the commissure attachment feature 316 shown in FIG. 10 is different from that shown in other figures, it is contemplated that either commissure attachment feature can be incorporated in any of the stents described above. The commissure attachment feature 316 as shown in FIG. 10 includes 4 smaller eyelets arranged in a two by two array, and an elongated eyelet oriented in the circumferential direction of the stent at one end of the commissure attachment feature. The commissure attachment feature of FIG. 10 also includes substantially straight and parallel sides, with a recess formed in each side. In a preferred embodiment, the recesses are aligned with the opposite ends of the elongated eyelet. The recesses are intended to facilitate suturing of the cuff and/or leaflets to the commissure attachment feature.

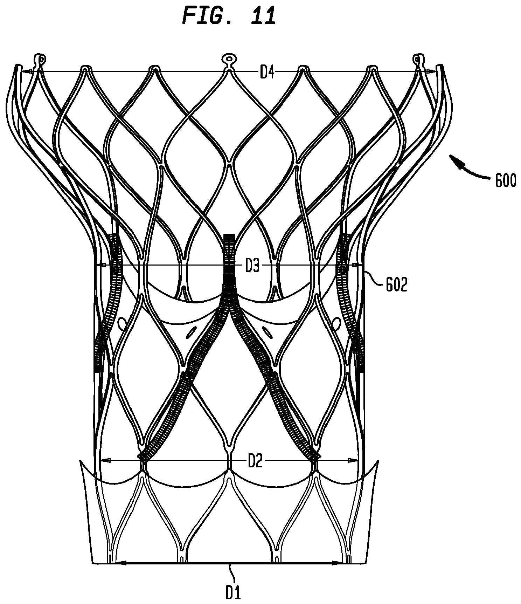

FIG. 11 is a side elevational view of another example of a collapsible prosthetic heart valve 600. As shown, collapsible prosthetic heart valve 600 may have a series of diameters at different positions along its length. In some examples, heart valve 600 may have a slight inward taper adjacent the annulus section such that diameter D1 at the inflow end of stent 602 is less than diameter D2 at approximately the longitudinal position at which each of the cells in the first annular row of cells connects to an adjacent cell in the row. In at least some examples, the diameter of prosthetic heart valve 600 is smallest at the inflow end of the annulus section (i.e., diameter D1) and largest at or near the outflow end of the aortic section (i.e., diameter D4). Diameter D1 may be substantially equal to diameter D3 at the transition (or waist) of prosthetic heart valve 600.

Although the invention herein has been described with reference to particular embodiments, it is to be understood that these embodiments are merely illustrative of the principles and applications of the present invention. For example, although the invention is described herein as applied to a prosthetic heart valve for replacing a native aortic valve, the invention is not so limited, and may be applied to prosthetic valves for replacing other types of cardiac valves. It will also be noted that while the inventions herein have been described predominately in terms of a tri-leaflet valve and a stent having a shape as illustrated in FIG. 3, the valve could be a bi-leaflet valve, such as the mitral valve, and the stent could have different shapes, such as a flared or conical annulus section, a less-bulbous aortic section, a differently shaped transition section, or any of the shapes described herein.

## Claims

1. A prosthetic heart valve (500) for replacing a native valve, the prosthetic heart valve comprising:
a stent (502) having an inflow end, an outflow end, are an annulus section adjacent the inflow end, an aortic section adjacent the outflow end, a transition section between the annulus section and the aortic section, a collapsed condition and an expanded condition, the stent including a first circumferential row of cells and a second circumferential row of cells positioned closer to the outflow end of the stent than the first circumferential row;
a first cuff (506) attached against a surface of the stent, the first cuff having an inflow edge and an outflow edge, the inflow edge being substantially straight, and the outflow edge including a series of first peaks extending a first distance toward the outflow end of the stent alternating with a series of second peaks extending a second distance toward the outflow end of the stent, the second distance being less than the first distance, the outflow edge of the first cuff being smoothly curved between each of the first peaks and each adjacent second peak so that a portion of the first cuff between the first peak and an adjacent second peak partially covers a cell in the second circumferential row;
a second cuff (507) having an inflow end and outflow end, the second cuff being located radially outward of the first cuff and radially outward of the stent, the inflow end of the second cuff having a substantially straight edge and the outflow end of the second cuff having an undulating edge; and
a valve assembly arranged radially inward of the first cuff.

2. The prosthetic heart valve (500) as claimed in claims 1, wherein the first circumferential row of cells is defined by a first plurality of struts and the second circumferential row of cells is defined by a second plurality of struts, the outflow edge of the first cuff (506) is folded over selected struts in the first circumferential row.

3. The prosthetic heart valve (500) as claimed in claim 2, wherein each of the second peaks of the first cuff (506) includes side portions, the side portions being folded over the selected struts in the first circumferential row of cells.

4. The prosthetic heart valve (500) as claimed in claim 2, wherein the outflow edge of the second cuff (507) includes a plurality of peaks alternating with a plurality of valleys, the plurality of peaks being connected to selected ones of the first plurality of struts at the intersection of adjacent cells in the first circumferential row.

5. The prosthetic heart valve (500) as claimed in claim 4, wherein the plurality of valleys are not connected to the stent (502) or to the first cuff (506).

## Patentansprüche

1. Herzklappenprothese (500) für den Ersatz einer nativen Klappe, wobei die Herzklappenprothese Folgendes umfasst:
einen Stent (502), der Folgendes aufweist: ein Einflussende, ein Ausflussende, einen Anulusabschnitt benachbart zum Einflussende, einen Aortenabschnitt benachbart zum Ausflussende, einen Übergangsabschnitt, zwischen dem Anulusabschnitt und dem Aortenabschnitt, einen zusammengeklappten Zustand und einen expandierten Zustand, wobei der Stent eine erste umlaufende Reihe von Zellen und eine zweite umlaufende Reihe von Zellen, die näher zu dem Ausflussende des Stents vorliegt als die erste umlaufende Reihe von Zellen, einschließt;
eine erste Manschette (506), die gegen eine Oberfläche des Stents angebracht ist, wobei die erste Manschette einen Einflussrand und einen Ausflussrand aufweist, wobei der Einflussrand im Wesentlichen gerade ist und der Ausflussrand eine Reihe von ersten Spitzen einschließt, die sich eine erste Entfernung in Richtung des Ausflussendes des Stents erstrecken, die sich mit einer Reihe von zweiten Spitzen abwechseln, die sich eine zweite Entfernung in Richtung des Ausflussendes des Stents erstrecken, wobei die zweite Entfernung geringer ist als die erste Entfernung, wobei der Ausflussrand der ersten Manschette zwischen jeder der ersten Spitzen und jeder benachbarten zweiten Spitze sanft gekrümmt ist, sodass ein Teil der ersten Manschette zwischen der ersten Spitze und einer benachbarten zweiten Spitze teilweise eine Zelle in der zweiten umlaufenden Reihe abdeckt;
eine zweite Manschette (507), die ein Einflussende und ein Ausflussende aufweist, wobei sich die zweite Manschette radial nach außen von der ersten Manschette und radial nach außen von dem Stent befindet, wobei das Einflussende der zweiten Manschette einen im Wesentlichen geraden Rand aufweist und das Ausflussende der zweiten Manschette einen welligen Rand aufweist; und
eine Klappenanordnung, die radial nach innen von der ersten Manschette angeordnet ist.

2. Herzklappenprothese (500) nach Anspruch 1, wobei die erste umlaufende Reihe von Zellen durch eine erste Vielzahl von Streben definiert ist und die zweite umlaufende Reihe von Zellen durch eine zweite Vielzahl von Streben definiert ist, wobei der Ausflussrand der ersten Manschette (506) über ausgewählte Streben in der ersten umlaufenden Reihe gefaltet ist.

3. Herzklappenprothese (500) nach Anspruch 2, wobei jede der zweiten Spitzen der ersten Manschette (506) Seitenteile einschließt, wobei die Seitenteile über die ausgewählten Streben in der ersten umlaufenden Reihe von Zellen gefaltet sind.

4. Herzklappenprothese (500) nach Anspruch 2, wobei der Ausflussrand der zweiten Manschette (507) eine Vielzahl von Spitzen einschließt, die sich mit einer Vielzahl von Tälern abwechseln, wobei die Vielzahl von Spitzen mit den ausgewählten der ersten Vielzahl von Streben an dem Kreuzungspunkt von benachbarten Zellen in der ersten umlaufenden Reihe verbunden sind.

5. Herzklappenprothese (500) nach Anspruch 4, wobei die Vielzahl von Tälern nicht mit dem Stent (502) oder mit der ersten Manschette (506) verbunden sind.

## Revendications

1. Valve cardiaque prothétique (500) pour remplacer une valve naturelle, la valve cardiaque prothétique comprenant :
un stent (502) ayant une extrémité d'entrée de flux, une extrémité de sortie de flux, et une section d'anneau adjacente à l'extrémité d'entrée de flux, une section aortique adjacente à l'extrémité de sortie de flux, une section de transition entre la section d'anneau et la section aortique, une condition affaissée et une condition dilatée, le stent comprenant une première rangée circonférentielle d'alvéoles et une deuxième rangée circonférentielle d'alvéoles positionnée plus près de l'extrémité de sortie de flux du stent que la première rangée circonférentielle d'alvéoles ;
une première manchette (506) attachée contre une surface du stent, la première manchette ayant un bord d'entrée de flux et un bord de sortie de flux, le bord d'entrée de flux étant sensiblement droit, et le bord de sortie de flux comprenant une série de premières pointes s'étendant sur une première distance vers l'extrémité de sortie de flux du stent alternant avec une série de deuxièmes pointes s'étendant sur une deuxième distance vers l'extrémité de sortie de flux du stent, la deuxième distance étant moindre que la première distance, le bord de sortie de flux de la première manchette étant à courbe lisse entre chacune des premières pointes et chaque deuxième pointe adjacente de telle sorte qu'une partie de la première manchette entre la première pointe et une deuxième pointe adjacente recouvre partiellement un alvéole dans la deuxième rangée circonférentielle ;
une deuxième manchette (507) ayant une extrémité d'entrée de flux et une extrémité de sortie de flux, la deuxième manchette étant située radialement vers l'extérieur de la première manchette et radialement vers l'extérieur du stent, l'extrémité d'entrée de flux de la deuxième manchette ayant un bord sensiblement droit et l'extrémité de sortie de flux de la deuxième manchette ayant un bord ondulé ; et
un ensemble de valve arrangé radialement vers l'intérieur de la première manchette.

2. Valve cardiaque prothétique (500) selon la revendication 1, dans laquelle la première rangée circonférentielle d'alvéoles est définie par une première pluralité d'étais et la deuxième rangée circonférentielle d'alvéoles est définie par une deuxième pluralité d'étais, le bord de sortie de flux de la première manchette (506) est replié sur des étais sélectionnés dans la première rangée circonférentielle.

3. Valve cardiaque prothétique (500) selon la revendication 2, dans laquelle chacune des deuxièmes pointes de la première manchette (506) comprend des parties latérales, les parties latérales étant repliées sur les étais sélectionnés dans la première rangée circonférentielle d'alvéoles.

4. Valve cardiaque prothétique (500) selon la revendication 2, dans laquelle le bord de sortie de flux de la deuxième manchette (507) comprend une pluralité de pointes alternant avec une pluralité de creux, la pluralité de pointes étant raccordées à des étais sélectionnés de la première pluralité d'étais à l'intersection d'alvéoles adjacents dans la première rangée circonférentielle.

5. Valve cardiaque prothétique (500) selon la revendication 4, dans laquelle la pluralité de creux ne sont pas raccordés au stent (502) ni à la première manchette (506).
